# EUROPEAN PATENT SPECIFICATION

(11) **EP 3 072 509 B1**
(45) Date of publication and mention of the grant of the patent: **03.05.2023**
(21) Application number: 15161166.2
(22) Date of filing: 26.03.2015
(51) Int. Cl.: A61K 31/045, A61K 31/60, A61K 31/661, A61K 33/04, A61K 33/18, A61K 33/30, A61K 36/185, A61P 17/02, A61P 31/02

(54) **Compositions for treating Mortellaro disease and methods of preparation thereof**
Zusammensetzungen zur Behandlung der Mortellaro-Krankheit und Verfahren zu ihrer Herstellung
Compositions pour le traitement de la maladie de Mortellaro et procédés de préparation de celles-ci

(43) Date of publication of application: 28.09.2016
(73) Proprietor: De Leeuw, Johannes Antonius Cornelis, 1562 GP Krommenie (NL)
(72) Inventor: De Leeuw, Johannes Antonius Cornelis, 1562 GP Krommenie (NL)
(74) Representative: Arnold & Siedsma

(56) References cited:
- EP-A1- 2 724 724
- WO-A1-2013/016257
- US-A- 5 780 064
- US-A1- 2012 271 251
- LAVEN ET AL: "Treatment strategies for digital dermatitis for the UK", VETERINARY JOURNAL,, vol. 171, no. 1, 1 January 2006 (2006-01-01), pages 79-88, XP005253432, BAILLIERE TINDALL, LONDON, GB ISSN: 1090-0233, DOI: 10.1016/J.TVJL.2004.08.009
- N. SCHULTZ ET AL: "Efficacy of salicylic acid in the treatment of digital dermatitis in dairy cattle", THE VETERINARY JOURNAL, vol. 198, no. 2, 1 November 2013 (2013-11-01), pages 518-523, XP055189796, ISSN: 1090-0233, DOI: 10.1016/j.tvjl.2013.09.002
- MENNO HOLZHAUER ET AL: "The effect of an acidified, ionized copper sulphate solution on digital dermatitis in dairy cows", THE VETERINARY JOURNAL, vol. 193, no. 3, 1 September 2012 (2012-09-01), pages 659-663, XP055189801, ISSN: 1090-0233, DOI: 10.1016/j.tvjl.2012.06.049
- A.C. SMITH ET AL: "Effect of a tea tree oil and organic acid footbath solution on digital dermatitis in dairy cows", JOURNAL OF DAIRY SCIENCE, vol. 97, no. 4, 1 April 2014 (2014-04-01), pages 2498-2501, XP055189792, ISSN: 0022-0302, DOI: 10.3168/jds.2013-6776
- anonymous: "Bovine Digital Dermatitis", Holstein UK , 5 May 2006 (2006-05-05), XP002739836, Retrieved from the Internet: URL:http://ukcows.com/HolsteinUK/publicweb /HealthWelfare/docs/Cow%20Health/Disease%2 0Summaries/Bovine%20Digital%20Dermatitis.p df [retrieved on 2015-05-19]
- A. GOMEZ ET AL: "A randomized trial to evaluate the effect of a trace mineral premix on the incidence of active digital dermatitis lesions in cattle", JOURNAL OF DAIRY SCIENCE, vol. 97, no. 10, 1 October 2014 (2014-10-01), pages 6211-6222, XP055190208, ISSN: 0022-0302, DOI: 10.3168/jds.2013-7879
- DATABASE WPI Week 201235 Thomson Scientific, London, GB; AN 2011-N92568 XP002739837, & KR 2011 0119483 A (BIOCARE CO LTD) 2 November 2011 (2011-11-02)
- Anonymous: "Healthy Hooves HH 101 - Technical Data Sheet", Pre-Treatment Solutions , March 2014 (2014-03), XP002739838, Retrieved from the Internet: URL:http://www.healthyhooves.eu/images/HH1 01-TDS.pdf [retrieved on 2015-05-20]
- Chin Mee Wong ET AL: "In Vitro Study of Wound Healing Potential in Black Pepper (Piper nigrum L.)", Pharmaceutical and Biosciences Journal, 11 August 2014 (2014-08-11), pages 05-09, XP055916354, DOI: 10.20510/ukjpb/2/i4/91104

## Description

The invention relates to a composition comprising nutmeg powder, salicylic acid, powdered zinc, is defined by claims 1-6 and iodine tincture, a volatile solvent, an acidic compound and powdered peppercorn. The invention further relates to a method for producing the composition.

In order to care for infections of the hooves of livestock, such as cows or sheep, it is known to apply foot baths with various compositions. Such foot baths are, especially when caring for already occurring infections, most often not successful, since the composition applied may contain antibiotics, which is undesirable, or may be removed easily from the leg. Such foot baths are also relatively expensive.

N. Schultz et al., 2013 discloses the efficacy of salicylic acid in the treatment of digital dermatitis in, The veterinarian Journal, 198,p.518-523, dairy cattle. The disclosed compositions are applied using bandages. In order to prevent the premature removal of such a composition, it is known to cover the wound, by the application of a bandage around the hoof. The bandage needs to be removed after healing of the wound.

An animal of livestock may react heavily or unpredictably to the process of application or removal of a bandage, which may impose danger to the person executing such a process.

It is an object of the invention to reduce of even obviate the above mentioned disadvantages.

This object is achieved with a composition according to claim 1

The composition is easily applicable to a wound since the minimum amounts of volatile solvent and the acidic compound, which are individual components, provide the composition with an ointment-like structure. It may however comprise more of the volatile solvent or acidic compound as required, for instance to increase its fluid character when required.

The volatile solvent is a solvent that will readily evaporate after application at standard pressure. It may comprise solvents such as acetone, alcohols such as denatured alcohol (spiritus), or solvents with similar volatility at similar conditions.

The acidic compound is a mixture of acids, comprising at least phosphoric acid and sulfuric acid. Preferably, such compound is a mixture of organic and anorganic acids, since such a combination of acids has been shown to enhance the function of the composition in total.

After application, the composition will adhere to the wound, either by the action of the acidic compound or by the action of adherence to the sanies of the wound, which may either or both act as a carrier for the other components, or by the body temperature and subsequent hardening of the composition to the wound. When applied, the composition will form a film on the wound to care for the wound.

The composition comprises salicylic acid, preferably as or similar to as provided by Brenntag, as well as the nutmeg powder, the powdered zinc and the antiseptic provide care for the wound.

The composition may comprise stabilizing agents, such as for instance Pangel FG from Tolsa Group, which increases the homogeneity of the composition, especially over time.

The composition may also comprise other additives common to such kind of compositions, such as, but not limited to preservatives, colorants, fragrances, et cetera.

In a preferred embodiment of the composition according to the invention, the weight ratio between nutmeg powder, the keratolytic, the powdered zinc, the antiseptic, the solvent and the acidic compound is approximately equal to 1 : 3 : 3 : 3 : 2 : ≥4, preferably 1 : 3 : 3 : 3 : 2 : 4.

A composition with this weight ratio has been shown in particular to result in a composition which is easily applied to a wound. Moreover, the ratio of the functional components in such a weight ratio has been shown in particular to enhance the process of caring for the wound.

The composition according to the invention comprise powdered peppercorn.

The addition of powdered peppercorn has shown to form a crust on the wound, thereby further protecting the wound, for instance from contamination. Because of its protection, it is no longer necessary to apply a bandage to protect the wound, thereby increasing the safety of the use of the composition in comparison to known compositions.

The composition according the invention comprises the weight ratio between nutmeg powder and peppercorn of approximately equal to 2 : 1.

A weight ratio of approximately 2 parts of nutmeg powder versus 1 parts results in the formation of a crust of sufficient size for protection, with a limited of risk of excess solid components which may release from the wound prematurely.

The composition according to the invention comprises iodine tincture.

The iodine tincture has been shown to provide enhanced wound caring in combination with the other components of the composition.

In again another preferred embodiment of the composition according to the invention, the components further comprise an adhesive.

The addition of an adhesive to the composition increases the attachment of the film layer created on the wound. Such adhesives may for instance comprise Laponite, such as Laponite from Keyser & Mackay, or similar adhesive agents. Other adhesives may also be added, as an alternative or in combination with any of the previously mentioned adhesives.

In another preferred embodiment of the composition according to the invention, the pH of the composition is less than or equal to 0.7, preferably less than or equal to 0.4.

It is preferred to choose the ratio between the components such that the pH is less than or equal to 0.7, preferably less than or equal to 0.4 since such an acidity has been shown to increase the effectivity of the composition in caring for the wound.

In yet another preferred embodiment of the composition according to the invention, the composition is a composition for use as a medicament.

The composition may for instance be a composition for use in the treatment of Mortellaro disease.

The composition may be used for use as a medicament, for instance for application on a wound of a livestock such as a cow or a sheep suffering from Mortellaro disease.

The salicylic acid will then allow the ingredients to penetrate the wound. The nutmeg powder and the powdered zinc have then been shown to provide a wound healing effect in combination with the other ingredients of the composition, whereas the antiseptic disinfects the wound. The object of the invention is further achieved with a method according to claim 6.

It has been found that the production of the composition according to the invention is preferably executed by mixing all liquid components, or at least those mentioned to a first mixture and by separately mixing all solid components, or at least those mentioned to a second mixture, except powdered zinc, followed by mixing these two mixtures, wherein powdered zinc is added to the mixture.

Separate mixing of the liquid and the solid components of the mixture increases the ease of mixing and thereby the ease of production. Addition of the zinc powder only after mixing the first mixture and the second mixture with each other reduces the chance of the formation of agglomerates due to a reaction between the keratolytic, such as salicylic acid, and powdered zinc.

### Example 1

50 grams of nutmeg powder, 25 grams of powdered black peppercorn, 150 grams of salicylic acid from Brenntag were mixed to create a first mixture.

150 grams of denatured alcohol, 100 grams of iodine tincture and 200 grams of Healthy Hooves HH 101 (undiluted) were mixed to create a second mixture.

The first mixture and the second mixture were added to each other while stirring the resulting mixture. 150 grams of powdered zinc were added to the resulting mixture.

A composition (A) with a structure of an ointment was obtained. Similar compositions (B and C, respectively) were obtained if either Digiderm Premix from Swetrade or Digiderm from Agrinnovations was used in the same amount instead of Healthy Hooves HH 101.

### Example 2

The composition A obtained from example 1 was applied on a wound of a hoof of a cow infected from Mortellaro disease. In four weeks, the composition A formed a protective layer on the wound. In addition, the infection proceeded from seriously infected to restored.

The composition A obtained from example 1 was also applied on a wound of a hoof of a cow suffering from wall necrosis. In four weeks, the composition A formed a protective layer on the wound. In addition, the infection proceeded from seriously infected to almost restored.

## Claims

1. Composition suitable for the treatment of Mortellaro disease, comprising a mixture of the following components:
- nutmeg powder from *Myristica fragrans;*
- salicylic acid;
- powdered zinc;
- an antiseptic, wherein the antiseptic comprises iodine tincture;
- at least one tenth by weight of the composition of at least a volatile solvent; and
- at least one fifth by weight of the composition of an acidic compound, comprising at least phosphoric acid and sulfuric acid;
wherein the components further comprise powdered peppercorn and wherein the weight ratio between nutmeg powder and peppercorn is 2 : 1.

2. Composition according to claim 1, wherein the weight ratio between nutmeg powder, the salicylic acid, the powdered zinc, the antiseptic, the solvent and the acidic compound is 1 : 3 : 3 : 3 : 2 : ≥4, preferably 1 : 3 : 3 : 3 : 2 : 4 .

3. Composition according to any one of the preceding claims, wherein the components further comprise an adhesive.

4. Composition according to any one of the preceding claims, wherein the pH of the composition is less than or equal to 0.7, preferably less than or equal to 0.4.

5. Composition according to any one of the preceding claims for use as a medicament.

6. Method for producing the composition according to any one of the claims 1 to 4, comprising the steps of:
- mixing all of the liquid components of the composition to create a first mixture;
- mixing all of the solid components of the composition, except powdered zinc to create a second mixture;
- mixing of the first and the second mixture; and
- adding the powdered zinc to the mixture of the first and the second mixture.

## Patentansprüche

1. Für die Behandlung der Mortellaro-Krankheit geeignete Zusammensetzung, welche eine Mischung darstellt, bestehend aus folgenden Komponenten:
- Muskatnusspulver aus Myristica fragrans;
- Salicylsäure:
- pulverisiertes Zink;
- ein Antiseptikum, wobei das Antiseptikum Jodtinktur umfasst;
- mindestens ein Zehntel, bezogen auf das Gewicht der Zusammensetzung, zumindest eines flüchtigen Lösungsmittels; und
- mindestens ein Fünftel, bezogen auf das Gewicht der Zusammensetzung, einer sauren Verbindung, die zumindest Phosphorsäure und Schwefelsäure umfasst;
wobei die Komponenten ferner pulverisierte Pfefferkörner umfassen, und wobei das Gewichtsverhältnis zwischen Muskatnusspulver und Pfefferkörnern 2:1 beträgt.

2. Zusammensetzung nach Anspruch 1, wobei das Gewichtsverhältnis zwischen Muskatnusspulver, der Salicylsäure, dem pulverisierten Zink, dem Antiseptikum, dem Lösungsmittel und der sauren Verbindung 1 : 3 : 3 : 3 : 2 : ≥ 4, vorzugsweise 1 : 3 : 3 : 3 : 2 : 4 beträgt.

3. Zusammensetzung nach einem der vorherigen Ansprüche, wobei die Komponenten außerdem einen Klebstoff enthalten.

4. Zusammensetzung nach einem der vorherigen Ansprüche, wobei der pH-Wert der Zusammensetzung weniger als oder gleich 0,7, vorzugsweise weniger als oder gleich 0,4 beträgt.

5. Zusammensetzung nach einem der vorherigen Ansprüche zur Verwendung als Arzneimittel.

6. Verfahren zur Herstellung der Zusammensetzung nach einem der Ansprüche 1 bis 4, aufweisend die folgenden Schritte:
- Mischen aller flüssigen Komponenten der Zusammensetzung, um eine erste Mischung zu erzeugen;
- Mischen aller festen Komponenten der Zusammensetzung, mit Ausnahme von pulverisiertem Zink, um eine zweite Mischung zu erzeugen;
- Mischen der ersten und der zweiten Mischung; und
- Zugeben des pulverisierten Zinks zu der Mischung aus der ersten und der zweiten Mischung.

## Revendications

1. Composition destinée au traitement de la maladie de Mortellaro, comprenant un mélange des composants suivants :
poudre de noix de muscade à partir de Myristica fragrans ;
acide salicylique ;
zinc en poudre ;
un antiseptique, dans lequel l'antiseptique comprend de la teinture d'iode ;
au moins un solvant volatil représentant au moins un dixième en poids de la composition ; et
un composé acide, représentant au moins un cinquième en poids de la composition, comprenant au moins de l'acide phosphorique et de l'acide sulfurique ;
dans laquelle les composants comprennent, en outre, du poivre en poudre et dans laquelle le rapport en poids entre la poudre de noix de muscade et le poivre est 2 : 1.

2. Composition selon la revendication 1, dans laquelle le rapport en poids entre la poudre de noix de muscade, l'acide salicylique, le zinc en poudre, l'antiseptique, le solvant et le composé acide est de 1 : 3 : 3 : 3 : 2 : ≥ 4, de préférence, de 1 : 3 : 3 : 3 : 2 : 4.

3. Composition selon l'une quelconque des revendications précédentes, dans laquelle les composants comprennent, en outre, un adhésif.

4. Composition selon l'une quelconque des revendications précédentes, dans laquelle le pH de la composition est inférieur ou égal à 0,7, de préférence, inférieur ou égal à 0,4.

5. Composition selon l'une quelconque des revendications précédentes, destinée à une utilisation en tant que médicament.

6. Procédé de production de la composition selon l'une quelconque des revendications 1 à 4, comprenant les étapes de :
mélange de tous les composants liquides de la composition afin de créer un premier mélange :
mélange de tous les composants solides de la composition, excepté le zinc en poudre afin de créer un second mélange ;
mélange du premier et du second mélange ; et
ajout du zinc en poudre au mélange du premier et du second mélange.
